# EUROPEAN PATENT APPLICATION

(11) **EP 4 450 492 A1**
(43) Date of publication of application: **23.10.2024**
(21) Application number: 22908005.6
(22) Date of filing: 16.12.2022
(51) Int. Cl.: C07D 401/06, A61K 31/454, A61P 35/00, A61P 29/00, A61P 37/00, A61P 43/00

(54) **NOVEL ACID ADDITION SALT AND CRYSTALLINE FORM OF (2R,3S)-2-(3-(4,5-DICHLORO-1H-BENZO[D]IMIDAZOL-1-YL)PROPYL)PIPERIDIN-3-OL**

(30) Priority: 17.12.2021 KR 20210182188
(71) Applicant: Daewoong Pharmaceutical Co., Ltd., Hwaseong-si, Gyeonggi-do 18623 (KR)
(72) Inventor: LEE, Joon-Hwan, Seoul 06170 (KR); CHO, Min Jae, Seoul 06170 (KR); YOON, Ji Sung, Seoul 06170 (KR)
(74) Representative: Germain Maureau
(86) International application number: PCT/KR2022/020591
(87) International publication number: WO 2023/113540

(57) **Abstract**

The present disclosure provides novel (2R, 3S)-2-(3-(4,5-dichloro-1H-benzo[d]imidazol-1-yl)propyl)piperidin-3-ol monohydrochloride, (2R, 3S)-2-(3-(4,5-dichloro-1H-benzo[d]imidazol-1-yl)propyl)piperidin-3-ol oxalate, (2R, 3S)-2-(3-(4,5-dichloro-1H-benzo[d]imidazol-1-yl)propyl)piperidin-3-ol maleate, (2R, 3S)-2-(3-(4,5-dichloro-1H-benzo[d]imidazol-1-yl)propyl)piperidin-3-ol palmitate, and crystalline form of (2R, 3S)-2-(3-(4,5-dichloro-1H-benzo[d]imidazol-1-yl)propyl)piperidin-3-ol monohydrochloride. These novel acid addition salts and crystalline forms have low hygroscopicity and excellent severe stability under high temperature and high humidity conditions, and thus can be used pharmaceutically.

## Description

### [TECHNICAL FIELD]

The present disclosure relates to a pharmaceutically acceptable acid addition salt and crystalline form of (2R, 3S)-2-(3-(4,5-dichloro-1H-benzo[d]imidazol-1-yl)propyl)piperidin-3-ol.

### [BACKGROUND ART]

The selection of pharmaceutically acceptable salts and their polymorphs is a very important step in the process of research and development of new drugs. This is because certain salt forms and their polymorphs can have a significant impact on the production process development of drug raw materials, and the design and formulation of the finished drug product.

Specifically, the type of salts and their polymorphs may affect recrystallization yield, process speed, and purity in the final stages of producing the drug raw material, that is, in the recrystallization and purification process. Particularly, the speed in the crystallization process may vary depending on the size or shape of crystals, and thus, the selection of salts and polymorphs is also important from the viewpoint of the productivity and production cost of the drug raw material.

Additionally, the addition of salts to certain drugs can be a means of changing the physiochemical and biological properties without modifying the chemical structure of the drug. This is because in pharmaceutical aspects, physicochemical properties such as hygroscopicity, stability, solubility, flowability of particles and dissolution rate are affected by the form of salts and their polymorphs. Thus, the form of salts and their polymorphs are factors that determine the production process, production and storage conditions, shelf life and the like of the finished drug product.

Therefore, there is a need for continuous research on the selection of salts and polymorphs that can exhibit better stability and better bioavailability in the preparation of specific drug formulations.

Meanwhile, the present inventors have found (2R,3S)-2-(3-(4,5-dichloro-1H-benzo[d]imidazol-1-yl)propyl)piperidin-3-ol as a novel active pharmaceutical substance capable of inhibiting the prolyl-tRNA synthetase (PRS) enzyme which is one of the aminoacyl-tRNA synthetase (ARS) family enzymes and serves to activate an amino acid for protein synthesis.

Such PRS is present in a multisynthetase complex (MSC) in the form of EPRS (Glutamyl-Prolyl-tRNA Synthetase). In particular, among various MSCs, EPRS functions as a translational silencer that suppresses the production of VEGF A (vascular endothelial growth factor A). In addition, it is reported that EPRS is closely related with various solid tumors (Nat. Rev. Cancer, 2011, 11, 708-718). Therefore, an active pharmaceutical substance of the (2R,3S)-2-(3-(4,5-dichloro-1H-benzo[d]imidazol-1-yl)propyl)piperidin-3-ol can be usefully applied as a PRS (prolyl-tRNA synthetase) anti-fibrotic agent, an anti-inflammatory agent, an autoimmune therapeutic agent, or a next-generation anti-cancer agent that can be used alone or in combination with an existing target anti-cancer agent.

Therefore, the present inventors have conducted intensive research on the selection of pharmaceutically acceptable salts and their polymorphs that can improve the bioavailability and stability of (2R, 3S)-2-(3-(4,5-dichloro-1H-benzo[d]imidazol-1-yl)propyl)piperidin-3-ol, and as a result, found that when produced in the form of a specific acid addition salt, it can reduce hygroscopicity and improve stability under severe conditions, and completed the present disclosure.

### [DETAILED DESCRIPTION OF THE INVENTION]

### [Technical Problem]

It is an object of the present disclosure to provide a pharmaceutically acceptable acid addition salt of (2R, 3S)-2-(3-(4,5-dichloro-1H-benzo[d]imidazol-1-yl)propyl)piperidin-3-ol having low hygroscopicity and excellent stability.

It is another object of the present disclosure to provide a crystalline form of a pharmaceutically acceptable acid addition salt of (2R, 3S)-2-(3-(4,5-dichloro-1H-benzo[d]imidazol-1-yl)propyl)piperidin-3-ol having low hygroscopicity and excellent stability.

### [Technical Solution]

In order to achieve the above object, according to one aspect of the present disclosure,
there is provided (2R, 3S)-2-(3-(4,5-dichloro-1H-benzo[d]imidazol-1-yl)propyl)piperidin-3-ol monohydrochloride.

In another aspect of the present disclosure,
there is provided an acid addition salt of (2R, 3S)-2-(3-(4,5-dichloro-1H-benzo[d]imidazol-1-yl)propyl)piperidin-3-ol, wherein the acid addition salt is oxalate, maleate, or palmitate.

In yet another aspect of the present disclosure,
there is provided a crystalline form of (2R, 3S)-2-(3-(4,5-dichloro-1H-benzo[d]imidazol-1-yl)propyl)piperidin-3-ol monohydrochloride.

Meanwhile, (2R, 3S)-2-(3-(4,5-dichloro-1H-benzo[d]imidazol-1-yl)propyl)piperidin-3-ol, which is a novel active drug material, is represented by the following Chemical Formula 1:

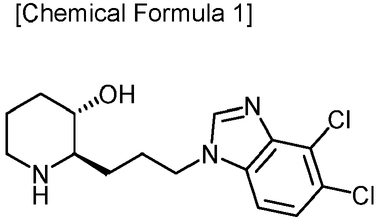

The (2R, 3S)-2-(3-(4,5-dichloro-1H-benzo[d]imidazol-1-yl)propyl)piperidin-3-ol and pharmaceutically acceptable salts thereof can inhibit PRS enzymatic activity while exhibiting reduced toxicity, and can thereby be used for preventing or treating diseases caused by abnormal PRS activity. Therefore, the (2R, 3S)-2-(3-(4,5-dichloro-1H-benzo[d]imidazol-1-yl)propyl)piperidin-3-ol and pharmaceutically acceptable salts thereof can be usefully applied to the prevention or treatment of cancer, inflammatory disease, autoimmune disease, or fibrosis caused by abnormal PRS activity.

Wherein, the (2R, 3S)-2-(3-(4,5-dichloro-1H-benzo[d]imidazol-1-yl)propyl)piperidin-3-ol and pharmaceutically acceptable salts thereof are an acid addition salt of (2R, 3S)-2-(3-(4,5-dichloro-1H-benzo[d]imidazol-1-yl)propyl)piperidin-3-ol. Such an acid addition salt can be prepared by reacting a free base of (2R, 3S)-2-(3-(4,5-dichloro-1H-benzo[d]imidazol-1-yl)propyl)piperidin-3-ol ) and an acid to be added in a specific ratio.

Also, the acid addition salt of (2R, 3S)-2-(3-(4,5-dichloro-1H-benzo[d]imidazol-1 -yl)propyl)piperidin-3-ol may show a crystalline form. The crystalline form of the acid addition salt of (2R, 3S)-2-(3-(4,5-dichloro-1H-benzo[d]imidazol-1-yl)propyl)piperidin-3-ol can be prepared by various crystallization methods such as evaporative crystallization, drawing-out crystallization, reaction crystallization, solvent-mediated polymorphism, and solid-state polymorphism, which are selected according to the thermodynamic and dynamic properties of the salt.

Wherein, the crystalline form of the acid addition salt of (2R, 3S)-2-(3-(4,5-dichloro-1H-benzo[d]imidazol-1-yl)propyl)piperidin-3-ol can be confirmed through X-ray powder diffraction analysis. Specifically, the crystalline form can be distinguished through a diffraction angle (2θ) showing characteristic peaks in an X-ray powder diffraction pattern and the intensity of the peaks according to each diffraction angle (2θ). Here, the diffraction angle (2θ) may be varied by ±0.2°, or preferably ±0.1°, depending on various factors such as the production technique of the sample for measurement, the procedure for fixing the sample for measurement, and the measurement instrument.

Further, the acid addition salt of (2R, 3S)-2-(3-(4,5-dichloro-1H-benzo[d]imidazol-1-yl)propyl)piperidin-3-ol and its crystalline form can also be distinguished through the endothermic onset temperature and the endothermic temperature showing the maximum endothermic peak in differential scanning calorimetry analysis, respectively. Here, the temperature can be varied by ±3°C, preferably ±2°C, more preferably ±1°C, depending on various factors such as the production technique of the sample for measurement, the measuring equipment, and the rate of temperature change.

Now, the present disclosure will be described in detail.

### Monohydrochloride, oxalate, maleate and palmitate of (2R, 3S)-2-(3-(4,5-dichloro-1H-benzo[d]imidazol-1-yl)propyl)piperidin-3-ol

On the other hand, the free base (free form) of (2R, 3S)-2-(3-(4,5-dichloro-1H-benzo[d]imidazol-1-yl) propyl) piperidin-3-ol is known to have poor stability. Therefore, in order to improve the stability of these compounds, the (2R, 3S)-2-(3-(4,5-dichloro-1H-benzo[d]imidazol-1-yl)propyl)piperidin-3-ol dihydrochloride has been studied as an acid addition salt of (2R, 3S)-2-(3-(4,5-dichloro-1H-benzo[d]imidazol-1-yl)propyl)piperidin-3-ol.

However, the (2R, 3S)-2-(3-(4,5-dichloro-1H-benzo[d]imidazol-1-yl)propyl)piperidin-3-ol dihydrochloride has high hygroscopicity, which was not preferable to produce it as a pharmaceutical product. Thus, the present inventors have conducted intensive research on acid addition salts that can improve the stability of the compound while having low hygroscopicity, and as a result, confirmed that when the above compound and a specific type of acid are combined in a specific equivalent ratio, hygroscopicity can be lowered and stability under severe conditions can be improved.

Specifically, the acid addition salt of the preferred (2R, 3S)-2-(3-(4,5-dichloro-1H-benzo[d]imidazol-1-yl)propyl)piperidin-3-ol is (2R, 3S)-2-(3-(4,5-dichloro-1H-benzo[d]imidazol-1-yl)propyl)piperidin-3-ol monohydrochloride.

Since the (2R, 3S)-2-(3-(4,5-dichloro-1H-benzo[d]imidazol-1-yl)propyl)piperidin-3-ol monohydrochloride as described above has fewer hydrogen ions and chlorine ions that can be ionized, and thus reduces bonds with moisture present in the air as compared to the dihydrochloride, it can exhibit low hygroscopicity and excellent stability under severe conditions relative to the (2R, 3S)-2-(3-(4,5-dichloro-1H-benzo[d]imidazol-1-yl)propyl)piperidin-3-ol dihydrochloride.

And, the (2R, 3S)-2-(3-(4,5-dichloro-1H-benzo[d]imidazol-1-yl)propyl)piperidin-3-ol monohydrochloride may have an endothermic onset temperature of 279.68 ± 3°C and show a maximum endothermic peak at an endothermic temperature of 281.04 ± 3°C in differential scanning calorimetry analysis. On the other hand, the (2R, 3S)-2-(3-(4,5-dichloro-1H-benzo[d]imidazol-1-yl)propyl)piperidin-3-ol dihydrochloride shows two endothermic peaks in differential scanning calorimetry analysis (a first peak having an endothermic onset temperature of 233.09°C and an endothermic temperature of the maximum endothermic peak within the peak of 242.09°C and a second peak having an endothermic onset temperature of 271.95°C and an endothermic peak of 275.07°C), which can be confirmed in Test Example 2 described later. Therefore, it can be seen that the (2R, 3S)-2-(3-(4,5-dichloro-1H-benzo[d]imidazol-1-yl)propyl)piperidin-3-ol monohydrochloride has higher stability against heat than the dihydrochloride.

Further, the (2R, 3S)-2-(3-(4,5-dichloro-1H-benzo[d]imidazol-1-yl)propyl)piperidin-3-ol monohydrochloride may be a crystalline form. The crystalline form of the monohydrochloride is advantageous from viewpoints of improvements in stability and hygroscopicity compared to an amorphous form or a mixture of crystalline form and amorphous form.

Further, preferred acid addition salt of (2R, 3S)-2-(3-(4,5-dichloro-1H-benzo[d]imidazol-1-yl)propyl)piperidin-3-ol is as follows:
(2R, 3S)-2-(3-(4,5-dichloro-1H-benzo[d]imidazol-1 -yl)propyl)piperidin-3-ol oxalate;
(2R, 3S)-2-(3-(4,5-dichloro-1H-benzo[d]imidazol-1 -yl)propyl)piperidin-3-ol maleate; and
(2R, 3S)-2-(3-(4,5-dichloro-1H-benzo[d]imidazol-1 -yl)propyl)piperidin-3-ol palmitate.

At this time, from the viewpoint of improving hygroscopicity, the acid addition salt is more preferably dioxalate, hemi-oxalate, hemi-maleate, or monopalmitate.

In one embodiment, the acid addition salt of (2R, 3S)-2-(3-(4,5-dichloro-1H-benzo[d]imidazol-1-yl)propyl)piperidin-3-ol is dioxalate. The dioxalate can be produced as oxalates I and II, depending on its preparation method.

Specifically, the dioxalate, that is, dioxalate I, may have an endothermic onset temperature of 210.42 ±3°C and show a maximum endothermic peak at an endothermic temperature of 213.66 ±3°C in differential scanning calorimetry analysis.

Specifically, the dioxalate, that is, dioxalate II, may have an endothermic onset temperature of 193.84 ±3°C and show a maximum endothermic peak at an endothermic temperature of 203.92 ±3°C in differential scanning calorimetry analysis.

In another embodiment, the acid addition salt of (2R, 3S)-2-(3-(4,5-dichloro-1H-benzo[d]imidazol-1-yl)propyl)piperidin-3-ol is hemi-oxalate.

The hemi-oxalate may have an endothermic onset temperature of 253.72 ±3°C and show a maximum endothermic peak at an endothermic temperature of 255.62 ±3°C in differential scanning calorimetry analysis.

In yet another embodiment, the acid addition salt of (2R, 3S)-2-(3-(4,5-dichloro-1H-benzo[d]imidazol-1-yl)propyl)piperidin-3-ol is hemi-maleate.

The hemi-maleate may have an endothermic onset temperature of 90.69 ±3°C and show a maximum endothermic peak at an endothermic temperature of 110.16 ±3°C in differential scanning calorimetry analysis.

In yet another embodiment, the palmitate may have an endothermic onset temperature of 92.32 ±3°C and show a maximum endothermic peak at an endothermic temperature of 98.59 ±3°C in differential scanning calorimetry analysis.

Meanwhile, the acid addition salt of (2R, 3S)-2-(3-(4,5-dichloro-1H-benzo[d]imidazol-1-yl)propyl)piperidin-3-ol can be prepared according to a preparation method comprising steps of:
1) mixing (2R, 3S)-2-(3-(4,5-dichloro-1H-benzo[d]imidazol-1-yl)propyl)piperidin-3-ol dihydrochloride with a base to prepare a free base of (2R, 3S)-2-(3-(4,5-dichloro-1H-benzo[d]imidazol-1-yl)propyl)piperidin-3-ol; and
2) mixing the free base of (2R, 3S)-2-(3-(4,5-dichloro-1H-benzo[d]imidazol-1-yl)propyl)piperidin-3-ol with an acid in the presence of an organic solvent and stirring the mixture.

Step 1 is a step of mixing (2R, 3S)-2-(3-(4,5-dichloro-1H-benzo[d]imidazol-1-yl)propyl)piperidin-3-ol dihydrochloride whose preparation method is known, with a base to prepare a free form from which hydrochloric acid has been removed, wherein the prepared free form exhibits a sticky liquid state.

At this time, as the base, at least one strong base selected from the group consisting of sodium hydroxide, lithium hydroxide and potassium hydroxide may be used.

Further, the pH in Step 1) is preferably about 9 to about 12 for removing hydrochloric acid.

Further, Step 2 is a step of mixing the free base of (2R, 3S)-2-(3-(4,5-dichloro-1H-benzo[d]imidazol-1-yl)propyl)piperidin-3-ol with an acid in the presence of an organic solvent and stirring the mixture to prepare a salt in which (2R, 3S)-2-(3-(4,5-dichloro-1H-benzo[d]imidazol-1-yl)propyl)piperidin-3-ol is chemically bonded with an acid.

In Step 2, the acid may be used by adjusting it according to the bonding ratio to be finally formed, relative to the 1 equivalent of the free base of (2R, 3S)-2-(3-(4,5-dichloro-1H-benzo[d]imidazol-1-yl)propyl)piperidin-3-ol. For example, when a monovalent acid addition salt in which the free base and a monovalent acid are combined is to be prepared, the acid may be used in an amount of 0.8 to 1.2 equivalents, preferably 1 equivalent, based on 1 equivalent of the free base. In addition, when a divalent acid addition salt in which the free base and a divalent acid are combined us to be prepared, the acid may be used in an amount of 1.8 to 2.2 equivalents, preferably 2 equivalents, based on 1 equivalent of the free base. Further, when a hemi-acid addition salt in which a divalent free base and a monovalent acid are combined is to be prepared, the acid may be used in an amount of 0.4 to 0.6 equivalents, preferably 0.5 equivalents, based on 1 equivalent of the free base.

Here, as the organic solvent, one or more selected from the group consisting of methanol, ethanol, acetonitrile, isopropanol, butanol, tetrahydrofuran, acetone, dimethylformamide and methylsulfoxide can be used. Such an organic solvent can be used in an amount (mL/g) of 5 to 60 times the weight of the free base of (2R, 3S)-2-(3-(4,5-dichloro-1H-benzo[d]imidazol-1-yl)propyl)piperidin-3-ol. Preferably, the organic solvent can be used in an amount of volume (mL/g) of 20 to 50 times, relative to the weight of the free base of (2R, 3S)-2-(3-(4,5-dichloro-1H-benzo[d]imidazol-1-yl)propyl)piperidin-3-ol.

Next, the step of stirring a mixed solution of the free base of (2R, 3S)-2-(3-(4,5-dichloro-1H-benzo[d]imidazol-1-yl)propyl)piperidin-3-ol, an acid and an organic solvent can be carried out at a temperature of 20°C to 30°C for 30 minutes to 30 hours. At this time, the stirring speed may be in the range of 50 to 300 rpm. A salt having high yield and high purity can be produced within the above range.

The acid addition salt of (2R, 3S)-2-(3-(4,5-dichloro-1H-benzo[d]imidazol-1-yl)propyl)piperidin-3-ol prepared by the above preparation method can be recovered from the solution by a vacuum filtration process. If necessary, the recovered acid addition salt may be washed and vacuum dried to obtain a high purity acid addition salt. In addition, reaction conditions such as the ratio of solvents, temperature range, process time, and the like described in the above preparation method can be adjusted according to the selected solvent.

Alternatively, the acid addition salt of (2R, 3S)-2-(3-(4,5-dichloro-1H-benzo[d]imidazol-1-yl)propyl)piperidin-3-ol, excluding hydrochloride, can also be prepared by the preparation method comprising Steps of:
1) mixing (2R, 3S)-2-(3-(4,5-dichloro-1H-benzo[d]imidazol-1-yl)propyl)piperidin-3-ol monochloride with a base to prepare a free base of (2R, 3S)-2-(3-(4,5-dichloro-1H-benzo[d]imidazol-1-yl)propyl)piperidin-3-ol; and
2) mixing the free base of (2R, 3S)-2-(3-(4,5-dichloro-1H-benzo[d]imidazol-1-yl)propyl)piperidin-3-ol with an acid other than hydrochloric acid in the presence of an organic solvent and stirring the mixture.

### Crystalline form of (2R, 3S)-2-(3-(4,5-dichloro-1H-benzo[d]imidazol-1-yl)propyl)piperidin-3-ol monohydrochloride

Meanwhile, the crystalline form of (2R, 3S)-2-(3-(4,5-dichloro-1H-benzo[d]imidazol-1-yl)propyl)piperidin-3-ol monohydrochloride may have peaks at diffraction angles (2θ±0.2°) of 15.0°, 17.2°, 19.8°, 23.6°, and 27.8° in an X-ray powder diffraction pattern.

Specifically, the crystalline form of (2R, 3S)-2-(3-(4,5-dichloro-1H-benzo[d]imidazol-1-yl)propyl)piperidin-3-ol monohydrochloride may have peaks at diffraction angles (2θ±0.2°) of 15.0°, 17.2°, 19.8°, 20.9°, 23.6°, 25.1°, 27.8°, 28.9° and 31.3° in an X-ray powder diffraction pattern.

Most specifically, the crystalline form of (2R, 3S)-2-(3-(4,5-dichloro-1H-benzo[d]imidazol-1-yl)propyl)piperidin-3-ol monohydrochloride may have peaks at diffraction angles (2θ±0.2°) of 7.5°, 12.5°, 15.0°, 16.5°, 17.2°, 18.9°, 19.8°, 20.9°, 22.3°, 23.6°, 24.8°, 25.1°, 26.1°, 27.8°, 28.9°, 30.1°, 30.9°, 31.3° and 32.4° in an X-ray powder diffraction pattern.

Further, the crystalline form of (2R, 3S)-2-(3-(4,5-dichloro-1H-benzo[d]imidazol-1-yl)propyl)piperidin-3-ol monohydrochloride may have an endothermic onset temperature of 279.68 ±3°C and show a maximum endothermic peak at the endothermic temperature of 281.04 ±3°C in differential scanning calorimetry analysis.

As the crystalline form of (2R, 3S)-2-(3-(4,5-dichloro-1H-benzo[d]imidazol-1-yl)propyl)piperidin-3-ol monohydrochloride shows a crystalline structure different from that of the crystalline form of (2R, 3S)-2-(3-(4,5-dichloro-1H-benzo[d]imidazol-1-yl)propyl)piperidin-3-ol dihydrochloride, has fewer hydrogen ions and chlorine ions that can be ionized, and thus reduces bonds with moisture present in the air, as compared to the dihydrochloride, it has a higher endothermic onset temperature than the crystalline form of the dihydrochloride, and thus can exhibit high thermal stability up to a relatively high temperature.

The crystalline form of (2R, 3S)-2-(3-(4,5-dichloro-1H-benzo[d]imidazol-1-yl)propyl)piperidin-3-ol monohydrochloride can be prepared using an evaporative crystallization or a drawing-out crystallization.

In one embodiment, the crystalline form of (2R, 3S)-2-(3-(4,5-dichloro-1H-benzo[d]imidazol-1-yl)propyl)piperidin-3-ol monohydrochloride can be prepared according to an evaporative crystallization method comprising Steps of:
1) dissolving or suspending (2R, 3S)-2-(3-(4,5-dichloro-1H-benzo[d]imidazol-1-yl)propyl)piperidin-3-ol monohydrochloride in at least one solvent selected from the group consisting of C₁₋₈ aliphatic alcohols, pentane, hexane, heptane, cyclohexane, benzene, toluene, methyl acetate, ethyl acetate, acetonitrile, acetone, tetrahydrofuran, dichloromethane, ethyl ether, ethylene glycol, propylene glycol and butylene glycol to prepare a solution or suspension; and
2) evaporating the solvent from the solution or suspension to crystallize the monohydrochloride.

Step 1 is a step of dissolving the monohydrochloride using a good solvent or a suspending solvent capable of completely dissolving the monohydrochloride, and can be carried out at 20°C to 30°C, preferably at room temperature. Further, the solvent may be used in an amount of volume (mL/g) 5 to 200 times, relative to the weight of the monohydrochloride.

At this time, as the C₁₋₈ aliphatic alcohol, methanol, ethanol, propanol, isopropanol, n-butanol, or n-octanol can be used.

Step 2 is a step of evaporating the solvent from the solution or suspension prepared in Step 1 and making the solution or suspension in a supersaturated state to crystallize monohydrochloride, and can be carried out at a temperature of 20°C to 30°C, preferably at room temperature for 1 to 4 days.

Alternatively, the crystalline form of (2R, 3S)-2-(3-(4,5-dichloro-1H-benzo[d]imidazol-1-yl)propyl)piperidin-3-ol monohydrochloride can be prepared according to a drawing-out crystallization method comprising Steps of:
1) dissolving (2R, 3S)-2-(3-(4,5-dichloro-1H-benzo[d]imidazol-1-yl)propyl)piperidin-3-ol monohydrochloride in a C₁₋₈ aliphatic alcohol solvent to prepare a solution; and
2) adding, to the solution, at least one crystallization solvent selected from the group consisting of C₁₋₈ aliphatic alcohol, pentane, hexane, heptane, cyclohexane, benzene, toluene, methyl acetate, ethyl acetate, acetonitrile, acetone, tetrahydrofuran, dichloromethane, ethyl ether, ethylene glycol, propylene glycol and butylene glycol and stirring the resulting mixture to crystallize the hydrochloride.

Step 1) is a step of dissolving the monohydrochloride using a good solvent capable of completely dissolving the monohydrochloride, and can be carried out at room temperature. Further, the solvent can be used in an amount of volume (mL/g) 5 to 200 times, relative to the weight of the monohydrochloride.

Step 2) is a step of adding an anti-solvent to the solution prepared in Step 1) and changing solubility to crystallize the hydrochloride, and this step can be carried out at a temperature of 20°C to 30°C for 1 to 4 days.

Further, the crystallization solvent can be used in an amount (mL/g) of 5 to 200 times the volume, or preferably in an amount of volume (mL/g) of 5 to 150 times, relative to the weight of the monohydrochloride, and the volume ratio between the solvent of Step 1) and the crystallization solvent of Step 2) may be 1:1 to 1:2. Within the above range, high-yield and high-purity crystals can be produced without economic loss due to an increase in crystal formation time and an excessive use of a solvent.

The crystal produced by the evaporative crystallization method or the drawing-out crystallization method can be recovered from solution by a vacuum filtration process. If necessary, the recovered crystals can be washed and vacuum dried to obtain a crystalline form of hydrochloride with high purity. In addition, reaction conditions such as the ratio of a solvent, temperature range, process time, and the like described in the above preparation methods may be adjusted according to the selected solvent.

Meanwhile, the present disclosure provides a pharmaceutical composition, comprising at least one selected from the group consisting of (2R, 3S)-2-(3-(4,5-dichloro-1H-benzo[d]imidazol-1-yl)propyl)piperidin-3-ol monohydrochloride, (2R, 3S)-2-(3-(4,5-dichloro-1H-benzo[d]imidazol-1-yl)propyl)piperidin-3-ol oxalate, (2R, 3S)-2-(3-(4,5-dichloro-1H-benzo[d]imidazol-1-yl)propyl)piperidin-3-ol maleate, (2R, 3S)-2-(3-(4,5-dichloro-1H-benzo[d]imidazol-1-yl)propyl)piperidin-3-ol palmitate, and a crystalline form of (2R, 3S)-2-(3-(4,5-dichloro-1H-benzo[d]imidazol-1-yl)propyl)piperidin-3-ol monohydrochloride.

More specifically, the present disclosure provides a pharmaceutical composition for preventing or treating cancer, inflammatory disease, autoimmune disease, or fibrosis, comprising at least one selected from the group consisting of (2R, 3S)-2-(3-(4,5-dichloro-1H-benzo[d]imidazol-1-yl)propyl)piperidin-3-ol monohydrochloride, (2R, 3S)-2-(3-(4,5-dichloro-1H-benzo[d]imidazol-1-yl)propyl)piperidin-3-ol oxalate, (2R, 3S)-2-(3-(4,5-dichloro-1H-benzo[d]imidazol-1-yl)propyl)piperidin-3-ol maleate, (2R, 3S)-2-(3-(4,5-dichloro-1H-benzo[d]imidazol-1-yl)propyl)piperidin-3-ol palmitate, and a crystalline form of (2R, 3S)-2-(3-(4,5-dichloro-1H-benzo[d]imidazol-1-yl)propyl)piperidin-3-ol monohydrochloride.

Such a pharmaceutical composition may include commonly used pharmaceutically acceptable carriers. The carriers are those usually used for the formulation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxy benzoate, propylhydroxybenzoate, talc, magnesium stearate, mineral oil, and the like, but are not limited thereto. The pharmaceutical composition may further include a lubricant, a wetting agent, a sweetening agent, a flavoring agent, an emulsifying agent, a suspending agent, a preserving agent, and the like, in addition to the above ingredients.

The pharmaceutical composition may be administered in an oral mode, or in a parenteral mode including intravenous, intramuscular, intraperitoneal, subcutaneous and transdermal administration.

At this time, the pharmaceutical composition may be administered in a therapeutically effective dosage, for example, an effective dosage of about 0.001 mg/kg to about 100 mg/kg per day. The dosage can be changed according to the formulation method, administration mode, age, body weight, venereal condition, food, administration time, administration route, excretion rate or reaction sensitivity of the patient.

The pharmaceutical composition of the present disclosure may be formulated into a preparation by using a pharmaceutically acceptable carrier according to a method easily practicable by those skilled in the art, to which the present disclosure pertains, such that the composition may be prepared in a unit dose form or prepared by being inserted into a multi-dose container. At this time, the preparation can be used without limitation as long as it is in a form suitable for pharmaceutical preparations, including oral formulations such as powders, granules, tablets, capsules, suspensions, emulsions, syrups and aerosols. external preparations such as ointments and creams, suppositories, sterile injection solutions, and the like. The preparation may further include dispersants or stabilizers.

### [ADVANTAGEOUS EFFECTS]

Novel acid addition salts and crystalline forms of (2R, 3S)-2-(3-(4,5-dichloro-1H-benzo[d]imidazol-1-yl)propyl)piperidin-3-ol according to the present disclosure have low hygroscopicity and excellent severe stability under high temperature and high humidity conditions, and thus can be used pharmaceutically.

### [BRIEF DESCRIPTION OF THE DRAWINGS]

FIG. 1 shows the results of differential scanning calorimetry analysis and thermogravimetric analysis of (2R, 3S)-2-(3-(4,5-dichloro-1H-benzo[d]imidazol-1-yl)propyl)piperidin-3-ol monohydrochloride prepared in Example 1-1.
FIG. 2 shows the results of differential scanning calorimetry analysis and thermogravimetric analysis of (2R, 3S)-2-(3-(4,5-dichloro-1H-benzo[d]imidazol-1-yl)propyl)piperidin-3-ol dioxalate I prepared in Example 1-2.
FIG. 3 shows the results of differential scanning calorimetry analysis and thermogravimetric analysis of (2R, 3S)-2-(3-(4,5-dichloro-1H-benzo[d]imidazol-1-yl)propyl)piperidin-3-ol dioxalate II prepared in Example 1-3.
FIG. 4 shows the results of differential scanning calorimetry analysis and thermogravimetric analysis of (2R, 3S)-2-(3-(4,5-dichloro-1H-benzo[d]imidazol-1-yl)propyl)piperidin-3-ol hemi-oxalate prepared in Example 1-4.
FIG. 5 shows the results of differential scanning calorimetry analysis and thermogravimetric analysis of (2R, 3S)-2-(3-(4,5-dichloro-1H-benzo[d]imidazol-1-yl)propyl)piperidin-3-ol hemi-maleate prepared in Example 1-5.
FIG. 6 shows the results of differential scanning calorimetry analysis and thermogravimetric analysis of (2R, 3S)-2-(3-(4,5-dichloro-1H-benzo[d]imidazol-1-yl)propyl)piperidin-3-ol palmitate prepared in Example 1-6.
FIG. 7 shows the results of differential scanning calorimetry analysis and thermogravimetric analysis of (2R, 3S)-2-(3-(4,5-dichloro-1H-benzo[d]imidazol-1-yl)propyl)piperidin-3-ol dihydrochloride prepared in Comparative Example 1.
FIG. 8 shows the results of differential scanning calorimetry analysis after 1 week of severe tests with respect to temperature of (2R, 3S)-2-(3-(4,5-dichloro-1H-benzo[d]imidazol-1-yl)propyl)piperidin-3-ol monohydrochloride prepared in Example 1-1.
FIG. 9 shows the results of differential scanning calorimetry analysis after 8 weeks of severe tests with respect to temperature of (2R, 3S)-2-(3-(4,5-dichloro-1H-benzo[d]imidazol-1-yl)propyl)piperidin-3-ol monohydrochloride prepared in Example 1-1.
FIG. 10 shows the results of differential scanning calorimetry analysis after 1 week of severe tests with respect to temperature of (2R, 3S)-2-(3-(4,5-dichloro-1H-benzo[d]imidazol-1-yl)propyl)piperidin-3-ol dihydrochloride prepared in Comparative Example 1.
FIG. 11 shows the results of differential scanning calorimetry analysis after 8 weeks of severe tests with respect to temperature of (2R, 3S)-2-(3-(4,5-dichloro-1H-benzo[d]imidazol-1-yl)propyl)piperidin-3-ol dihydrochloride prepared in Comparative Example 1.
FIG. 12 shows the X-ray powder diffraction pattern for the crystalline form of (2R, 3S)-2-(3-(4,5-dichloro-1H-benzo[d]imidazol-1-yl)propyl)piperidin-3-ol monohydrochloride prepared in Comparative Example 2-1.
FIG. 13 shows the X-ray powder diffraction pattern for the crystalline form of (2R, 3S)-2-(3-(4,5-dichloro-1H-benzo[d]imidazol-1-yl)propyl)piperidin-3-ol dihydrochloride prepared in Comparative Example 2.
FIG. 14 shows the results of differential scanning calorimetry analysis and thermogravimetric analysis of the crystalline form of (2R, 3S)-2-(3-(4,5-dichloro-1H-benzo[d]imidazol-1-yl)propyl)piperidin-3-ol monohydrochloride prepared in Example 2-1.

### [DETAILED DESCRIPTION OF THE EMBODIMENTS]

Hereinafter, preferred examples are presented to aid understanding of the present disclosure. However, the following examples are only provided for easier understanding of the present disclosure, and the contents of the present disclosure are not limited thereby.

### Preparation Example: Preparation of (2R, 3S)-2-(3-(4,5-dichloro-1H-benzo[d]imidazol-1-yl)propyl)piperidin-3-ol (free base)

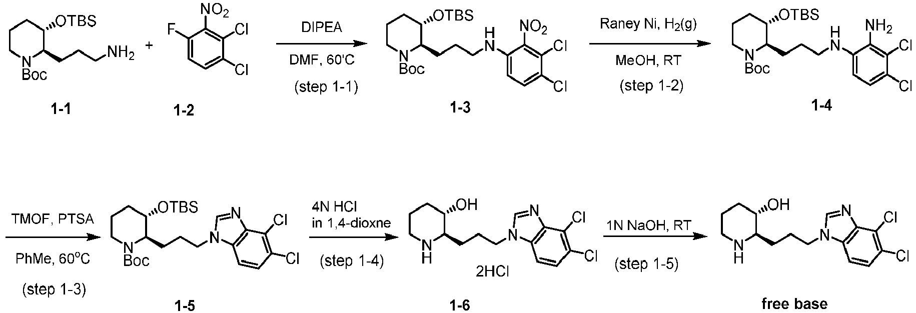

### Step 1-1) Preparation of Compound 1-3 (tert-butyl (2R,3S)-3-((tertbutyldimethylsilyl)oxy)-2-(3-((3,4-dichloro-2-nitrophenyl)amino)propyl)piperidine-1-carboxylate)

tert-Butyl(2R,3S)-2-(3-aminopropyl)-3-((tert-butyldimethylsilyl)oxy)piperidine-1-carboxylate (1.0 g, 2.7 mmol) as Compound 1-1 was added to and dissolved in N,N-dimethylformamide (25 mL, 0.67 M), which to 1,2-dichloro-4-fluoro-3-nitrobenzene (629.97 mg, 3.0 mmol) as Compound 1-2 and N,N-diisopropylethylamine (0.93 mL, 5.4 mmol) were added and then heated and stirred at 60°C for 2 hours. When the reaction was completed, the resulting mixture was diluted with ethyl acetate and washed with saturated sodium chloride solution. The organic layer was collected, dried over sodium sulfate, filtered and then concentrated under reduced pressure, and purified by column chromatography (hexane:ethyl acetate=5:1) to obtain Compound 1-3 (1.2 g, yield: 81%).

¹H NMR (500 MHz, MeOD): δ 7.43 (d, 1H), 6.89 (d, 1H), 4.08 (s, 1H), 3.94 (s, 1H), 3.76 (s, 1H), 3.24 (m, 2H), 2.76 (s, 1H), 1.86 (m, 1H), 1.74 (m, 2H), 1.58 (m, 3H), 1.48 (s, 10H), 1.45 (s, 1H), 0.90 (s, 9H), 0.07 (d, 6H)

### Step 1-2) Preparation of Compound 1-4 (tert-butyl(2R,3S)-2-(3-((2-amino-3,4-dichlorophenyl)amino)propyl)-3-((tert-butyldimethylsilyl)oxy)piperidine-1-carboxylate)

Compound 1-3 (2.6 mg, 4.9 mmol) obtained from Step 1-1 was dissolved in methanol (25 mL, 0.2 M), to which an appropriate amount of Raney nickel was added. After connecting a hydrogen balloon, the mixture was stirred at room temperature for 1 hour. When the reaction was completed, the reaction solution was filtered with a celite and concentrated under reduced pressure to obtain Compound 1-4. Subsequent reactions were carried out without purification procedure.

### Step 1-3) Preparation of Compound 1-5 (tert-butyl (2R,3S)-3-((tertbutyldimethylsilyl)oxy)-2-(3-(4,5-dichloro-1H-benzo[d]imidazol-1-yl)propyl)piperidine-1-carboxylate)

Compound 1-4 obtained from Step 1-2 was added to and dissolved in toluene (30 mL, 0.16 M), to which trimethylorthoformate (1.6 mL, 14.6 mmol) and paratoluenesulfonic acid (168 mg, 0.98 mmol) were added and then heated and stirred at 50°C for 6 hours. When the reaction was completed, the solvent was removed and the resulting mixture was diluted with ethyl acetate and washed with saturated sodium chloride solution. The organic layer was collected, dried over magnesium sulfate, filtered and concentrated under reduced pressure, and then purified by column chromatography (hexane:ethyl acetate=1:1) to obtain Compound 1-5 (1.8 g, yield: 74%).

¹H NMR (500 MHz, MeOD): δ 8.30 (s, 1H), 7.56 (d, 1H), 7.43 (d, 1H), 4.30 (m, 2H), 4.17 (s, 1H), 4.05 (s, 1H), 3.91 (d, 1H), 3.73 (s, 1H), 2.68 (s, 1H), 1.87 (s, 3H), 1.70 (t, 2H), 1.55 (d, 1H), 1.45 (m, 10H), 1.42 (s, 1H), 0.90 (s, 9H), 0.07 (d, 6H)

### Step 1-4) Preparation of (2R, 3S)-2-(3-(4,5-dichloro-1H-benzo[d]imidazol-1-yl)propyl)piperidin-3-ol dihydrochloride

Compound 1-5 (1.8 g, 3.6 mmol) obtained from Step 1-3 was dissolved in a small amount of tetrahydrofuran, to which 4N hydrogen chloride dioxane solution (30 mL, 0.12 M) was added and then stirred at room temperature for 12 hours. When the reaction is complete, the reaction solution was concentrated under reduced pressure to remove the solvent, dissolved in a small amount of methanol, and crystallized with diethyl ether to obtain Compound 1-6, i.e., (2R, 3S)-2-(3-(4,5-dichloro-1H-benzo[d]imidazol-1-yl)propyl)piperidin-3-ol 2HCl (26 mg, yield: 81%).

¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Step 1-5) Preparation of (2R, 3S)-2-(3-(4,5-dichloro-1H-benzo[d]imidazol-1-yl)propyl)piperidin-3-ol (free base)

200 mg of Compound 1-6 obtained from Step 1-4 was placed in a beaker, and dissolved in 2 mL of water. 1 mL of 1N NaOH was added thereto and stirred well, and then the mixture was placed in a separatory funnel, wherein the pH was about 9.5. This was extracted with methylene chloride (MC) and concentrated by rotary evaporation. Then, the residual solvent was removed under vacuum conditions to obtain (2R, 3S)-2-(3-(4,5-dichloro-1H-benzo[d]imidazol-1-yl)propyl)piperidin-3-ol in a sticky liquid state (150 mg, yield: 92%).

### Molecular weight: 328.24

¹H-NMR (500 MHz, DMSO): δ 8.39 (s, 1H). 7.65 (d, 1H), 7.46 (d, 1H), 4.47 (d, 1H), 4.25 (t, 2H), 2.85 (m, 1H), 2.76 (d, 1H), 2.28 (m, 1H), 2.07 (m, 1H), 1.95 (m, 1H), 1.82 (m, 1H), 1.73 (m, 2H), 1.50 (m, 1H), 1.27 (m, 1H), 1.12 (m, 2H)

In the Examples below, (2R, 3S)-2-(3-(4,5-dichloro-1H-benzo[d]imidazol-1-yl)propyl)piperidin-3-ol (free base) prepared in Preparation Example was used.

### Example 1-1: Preparation of (2R, 3S)-2-(3-(4,5-dichloro-1H-benzo[d]imidazol-1-yl)propyl)piperidin-3-ol monohydrochloride

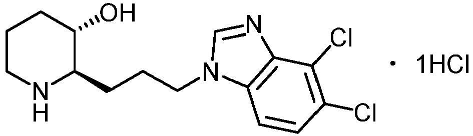

To 100 mg of (2R, 3S)-2-(3-(4,5-dichloro-1H-benzo[d]imidazol-1-yl)propyl)piperidin-3-ol (free base) prepared in the Preparation Example, 4 mL of ethanol was added and 32 mg (1 eq.) of 35% HCl solution was added, and then stirred at room temperature for 10 minutes. The resulting white solid was filtered and washed with ethyl acetate to obtain the title compound (103 mg, yield: 93%).

### Molecular weight 364.70

¹H-NMR (500 MHz, DMSO): δ 8.45 (s, 1H). 7.71 (d, 1H), 7.47 (d, 1H), 5.40 (d, 1H), 4.32 (m, 2H), 3.41 (m, 1H), 3.08 (d, 1H), 2.75 (m, 2H), 2.08 (m, 1H), 1.97 (m, 1H), 1.85 (m, 2H), 1.75 (m, 1H), 1.65 (m, 1H), 1.52 (m, 1H), 1.35 (m, 1H)

### Example 1-2: Preparation of (2R, 3S)-2-(3-(4,5-dichloro-1H-benzo[d]imidazol-1-yl)propyl)piperidin-3-ol dioxalate I

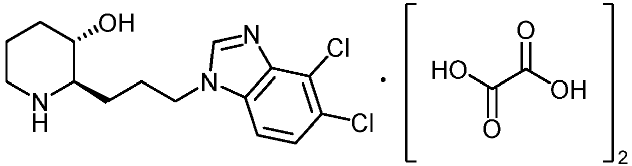

To 100 mg of (2R, 3S)-2-(3-(4,5-dichloro-1H-benzo[d]imidazol-1-yl)propyl)piperidin-3-ol (free base) prepared in the Preparation Example, 5 mL of methanol was added and 76.9 mg (2 eq.) of oxalic acid dehydrate was added and then stirred at room temperature for one day. The mixture was left at room temperature for one day until a solid was formed, and then the formed solid was washed to obtain the title compound (108 mg, yield: 70%).

### Molecular weight: 508.31

¹H-NMR (500 MHz, DMSO): δ 8.40 (s, 1H). 7.69 (d, 1H), 7.48 (d, 1H), 4.31 (m, 2H), 3.45 (m, 1H), 3.15 (m, 1H), 2.77 (m, 2H), 2.01 (m, 1H), 1.90 (m, 3H), 1.78 (m, 1H), 1.58 (m, 1H), 1.45 (m, 1H), 1.35 (m, 1H)

### Example 1-3: Preparation of (2R, 3S)-2-(3-(4,5-dichloro-1H-benzo[d]imidazol-1-yl)propyl)piperidin-3-ol dioxalate II

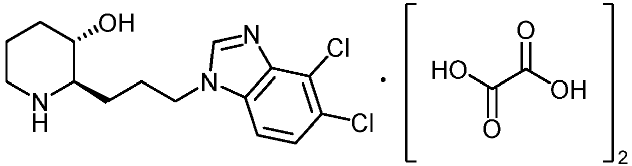

To 100 mg of (2R, 3S)-2-(3-(4,5-dichloro-1H-benzo[d]imidazol-1-yl)propyl)piperidin-3-ol (free base) prepared in the Preparation Example, 5 mL of acetonitrile was added and 76.9 mg (2 eq.) of oxalic acid dihydrate was added, and then stirred at room temperature for one day. The mixture was left at room temperature for one day until a solid was formed, and then the formed solid was washed to obtain the title compound (140 mg, yield: 90%).

### Molecular weight: 508.31

¹H-NMR (500 MHz, DMSO): δ 8.40 (s, 1H). 7.69 (d, 1H), 7.48 (d, 1H), 4.32 (m, 2H), 3.36 (m, 1H), 3.11 (d, 1H), 2.77 (m, 2H), 1.92 (m, 1H), 1.89 (m, 3H), 1.77 (m, 1H), 1.58 (m, 1H), 1.47 (m, 1H), 1.35 (m, 1H)

### Example 1-4: Preparation of (2R, 3S)-2-(3-(4,5-dichloro-1H-benzo[d]imidazol-1-yl)propyl)piperidin-3-ol hemi-oxalate

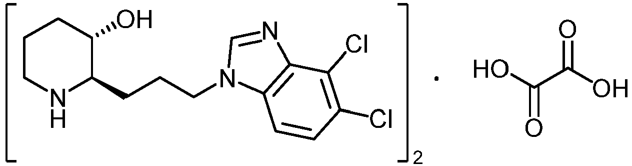

102.2 mg of (2R, 3S)-2-(3-(4,5-dichloro-1H-benzo[d]imidazol-1-yl)propyl)piperidin-3-ol (free base) prepared in the Preparation Example was placed in a round bottom flask, and completely dissolved in 5 mL of methanol. 19.6 mg (0.5 eq.) of oxalic acid dihydrate was placed in a flask in which the free base was dissolved, and then stirred for 2 hours. The result was dried in the open air at room temperature to obtain the title compound as a solid (84 mg, yield: 74%).

### Molecular weight: 373.26

¹H-NMR (500 MHz, DMSO): δ 8.40 (s, 1H). 7.69 (d, 1H), 7.47 (d, 1H), 4.30 (m, 2H), 3.22 (m, 1H), 3.03 (d, 1H), 2.60 (m, 2H), 2.02 (m, 1H), 1.88 (m, 2H), 1.80 (m, 1H), 1.69 (m, 1H), 1.50 (m, 1H), 1.38 (m, 1H), 1.29 (m, 1H)

### Example 1-5: Preparation of (2R, 3S)-2-(3-(4,5-dichloro-1H-benzo[d]imidazol-1-yl)propyl)piperidin-3-ol hemi-maleate

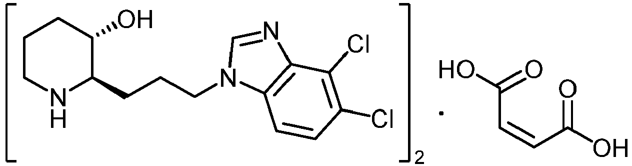

100 mg of (2R, 3S)-2-(3-(4,5-dichloro-1H-benzo[d]imidazol-1-yl)propyl)piperidin-3-ol (free base) prepared in the Preparation Example was placed in a round bottom flask, and completely dissolved in 2.5 mL of methanol. 20.4 mg (0.5 eq.) of maleic acid was placed in a flask in which the free base was dissolved, and then stirred for 2 hours. Then, after methanol was blown off, acetonitrile was added and stirred for 4 hours, and then dried in the open air at room temperature to obtain the title compound as a solid (88 mg, yield: 74.5%).

### Molecular weight: 386.28

¹H-NMR (500 MHz, DMSO): δ 8.40 (s, 1H). 7.68 (d, 1H), 7.49 (d, 1H), 6.01 (s, 1H), 5.07 (s, 1H), 4.30 (m, 2H), 3.17 (s, 1H), 2.98 (d, 1H), 2.62 (m, 2H), 1.98 (m, 1H), 1.80 (m, 3H), 1.69 (m, 1H), 1.45 (m, 1H), 1.29 (m, 2H)

### Example 1-6: Preparation of (2R, 3S)-2-(3-(4,5-dichloro-1H-benzo[d]imidazol-1-yl)propyl)piperidin-3-ol palmitate

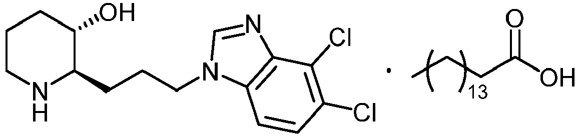

100 mg of (2R, 3S)-2-(3-(4,5-dichloro-1H-benzo[d]imidazol-1-yl)propyl)piperidin-3-ol (free base) prepared in the Preparation Example was placed in a vial and completely dissolved in 4.8 mL of ethanol. 78.7 mg (1 eq.) of palmitic acid was placed in the vial in which the free base is dissolved, and then stirred for 2 hours. Then, after ethanol was blown off, ethyl acetate was added and stirred for 4 hours, and then dried in the open air at room temperature to obtain the title compound as a solid (154 mg, yield: 86.5%).

### Molecular weight: 584.67

¹H-NMR (500 MHz, DMSO): δ 8.39 (s, 1H). 7.66 (d, 1H), 7.46 (d, 1H), 4.26 (m, 2H), 2.90 (m, 1H), 2.79 (d, 1H), 2.31 (m, 1H), 2.15 (m, 3H), 1.98 (m, 1H), 1.78 (m, 3H), 1.52 (m, 1H), 1.46 (m, 2H), 1.28 (m, 1H), 1.23 (m, 24H), 1.13 (m, 2H), 0.85 (m, 3H)

### Comparative Example 1: Preparation of (2R, 3S)-2-(3-(4,5-dichloro-1H-benzo[d]imidazol-1-yl)propyl)piperidin-3-ol dihydrochloride

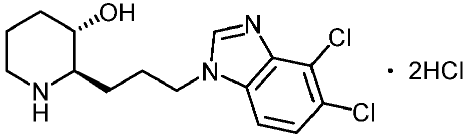

Steps 1-1 to 1-4 were carried out the same manner as in Preparation Example to obtain the title (2R, 3S)-2-(3-(4,5-dichloro-1H-benzo[d]imidazol-1-yl)propyl)piperidin-3-ol 2HCl (26 mg, yield: 81%)

### Molecular weight: 401.15

¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Test Example 1: PRS Enzyme Activity Inhibition Experiment

IC₅₀ values of PRS enzyme activity inhibition of the (2R, 3S)-2-(3-(4,5-dichloro-1H-benzo[d]imidazol-1-yl)propyl)piperidin-3-ol monohydrochloride prepared in Example 1-1 were measured as follows.

Specifically, the portion corresponding to PRS in cDNA of EPRS was subcloned, and the obtained high-purity PRS protein was purified and used in the experiment. The compounds (1 µM) prepared in Examples were added into the reaction buffer (20 mM KPO₄ (pH 7.4), 6 mM MgAc, 5 mM ATP, 400 mg/mL tRNA, 0.5 mM DTT, 20 mCi[3H]proline (1 mCi/mL)) and allowed to react at 37°C for 5 to 10 minutes. The reaction was terminated with 3M paper that was in advance dried by addition of 5% TCA. The radioactivity was measured using a liquid scintillation counter. % Inhibition and IC₅₀ values of the compounds were calculated and analyzed using Microsoft Excel or Sigma Plot 8.0. As a result, (2R, 3S)-2-(3-(4,5-dichloro-1H-benzo[d]imidazol-1-yl)propyl)piperidin-3-ol monohydrochloride showed a value of 74 nM.

Thus, it can be seen that (2R, 3S)-2-(3-(4,5-dichloro-1H-benzo[d]imidazol-1-yl)propyl)piperidin-3-ol monohydrochloride inhibits PRS enzyme activity and thus can be used in pharmaceutical compositions for preventing or treating diseases caused by abnormal PRS activity.

### Test Example 2: Differential scanning calorimetry and thermogravimetric analysis of acid addition salts

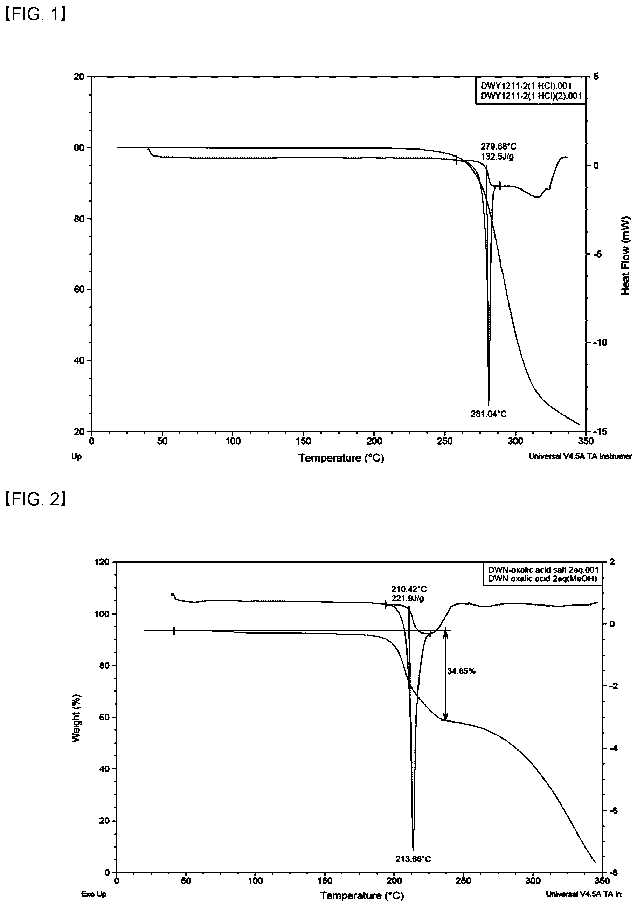

The acid addition salts prepared in Examples 1-1 to 1-6 and Comparative Example 1 were subjected to differential scanning calorimetry analysis and thermogravimetric analysis, and the results are also shown in FIGS. 1 to 7, respectively. At this time, the differential scanning calorimetry analysis was performed using a differential scanning calorimeter (DSC3, manufactured by Mettler-Toledo) in a tightly closed pan under nitrogen gas at a scan rate of 10°C/min while raising the temperature from 20°C to 350°C. In addition, the thermogravimetric analysis (TGA) was performed using a thermogravimetric analyzer (Q50, manufactured by TA Instruments, U.S.A.) at a scan rate of 10°C/min under nitrogen gas while raising the temperature from 20°C to 350°C.

Referring to FIG. 1, it can be confirmed that (2R, 3S)-2-(3-(4,5-dichloro-1H-benzo[d]imidazol-1-yl)propyl)piperidin-3-ol monohydrochloride prepared in Example 1-1 has an endothermic onset temperature of 279.68°C and shows a maximum endothermic peak at the endothermic temperature of 281.04°C in differential scanning calorimetry analysis. In addition, residual solvent and moisture were not confirmed by thermogravimetric analysis.

Referring to FIG. 2, it can be confirmed that (2R, 3S)-2-(3-(4,5-dichloro-1H-benzo[d]imidazol-1-yl)propyl)piperidin-3-ol dioxalate I prepared in Example 1-2 has an endothermic onset temperature of 210.42°C and shows a maximum endothermic peak at the endothermic temperature of 213.66°C in differential scanning calorimetry analysis.

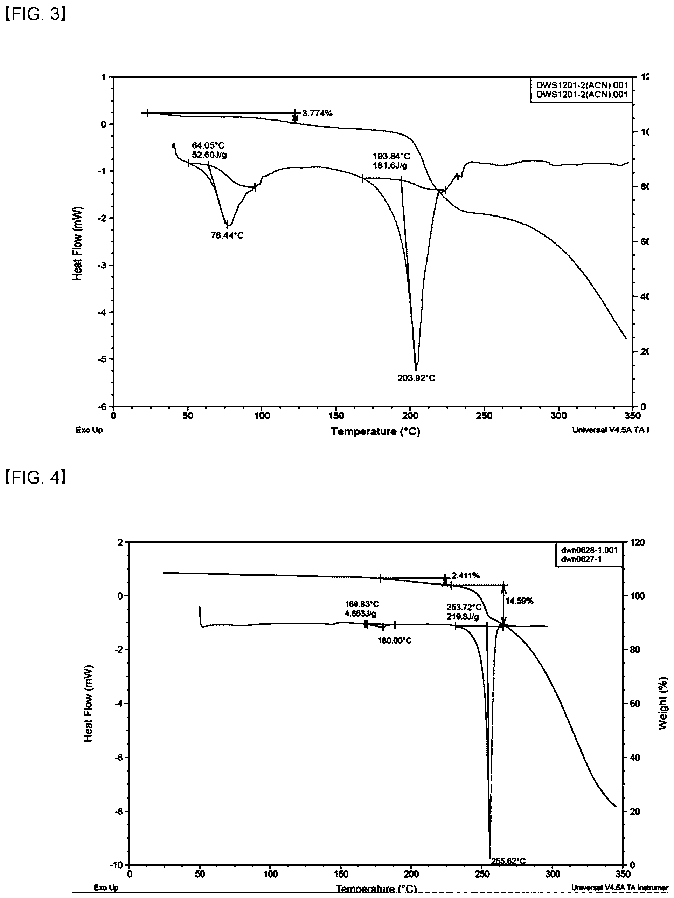

Referring to FIG. 3, it can be confirmed that (2R, 3S)-2-(3-(4,5-dichloro-1H-benzo[d]imidazol-1-yl)propyl)piperidin-3-ol dioxalate II prepared in Example 1-3 has an endothermic onset temperature of 193.84°C and shows a maximum endothermic peak at the endothermic temperature of 203.92°C in differential scanning calorimetry analysis. However, as a result of confirming the peak shown at 64°C in FIG. 3 with a hot stage microscope (HSM, BA310Pol, manufactured by Motic), it is determined that the peak is due to residual solvent and not moisture.

Referring to FIG. 4, it can be confirmed that (2R, 3S)-2-(3-(4,5-dichloro-1H-benzo[d]imidazol-1-yl)propyl)piperidin-3-ol hemi-oxalate prepared in Example 1-4 has an endothermic onset temperature of 253.72°C and shows a maximum endothermic peak at the endothermic temperature of 255.62°C in differential scanning calorimetry analysis. Further, as a result of thermogravimetric analysis, it can be seen that the compound melts and decomposes as the temperature rises.

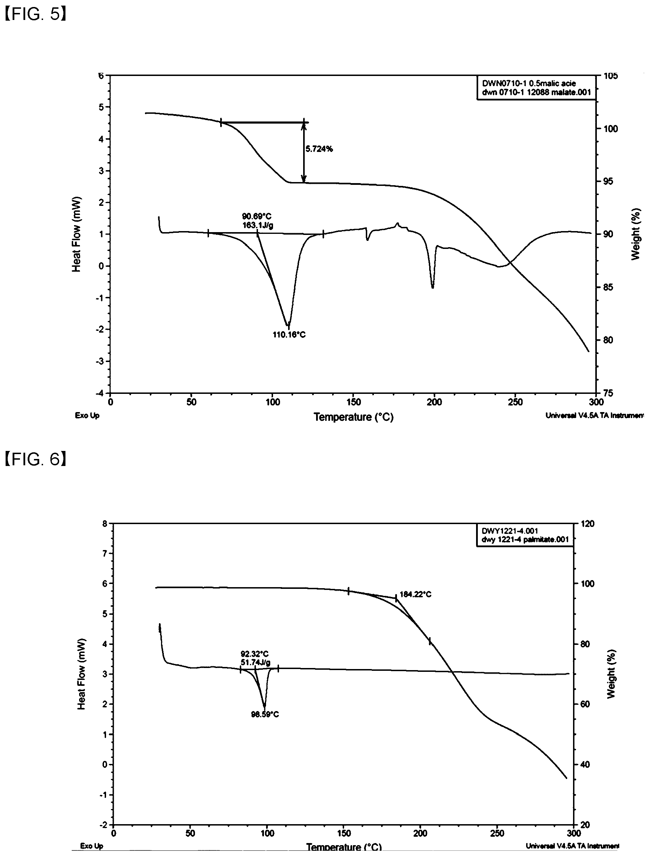

Referring to FIG. 5, it can be confirmed that (2R, 3S)-2-(3-(4,5-dichloro-1H-benzo[d]imidazol-1-yl)propyl)piperidin-3-ol hemi-maleate prepared in Example 1-5 has an endothermic onset temperature of 90.69°C and shows a maximum endothermic peak at the endothermic temperature of 110.16°C in differential scanning calorimetry analysis. Further, as a result of thermogravimetric analysis, it can be seen that the compound melts and decomposes as the temperature rises.

Referring to FIG. 6, it can be confirmed that (2R, 3S)-2-(3-(4,5-dichloro-1H-benzo[d]imidazol-1-yl)propyl)piperidin-3-ol palmitate prepared in Example 1-6 has an endothermic onset temperature of 92.32°C and shows a maximum endothermic peak at the endothermic temperature of 98.59°C in differential scanning calorimetry analysis. Further, as a result of thermogravimetric analysis, it can be seen that the compound melts and decomposes as the temperature rises.

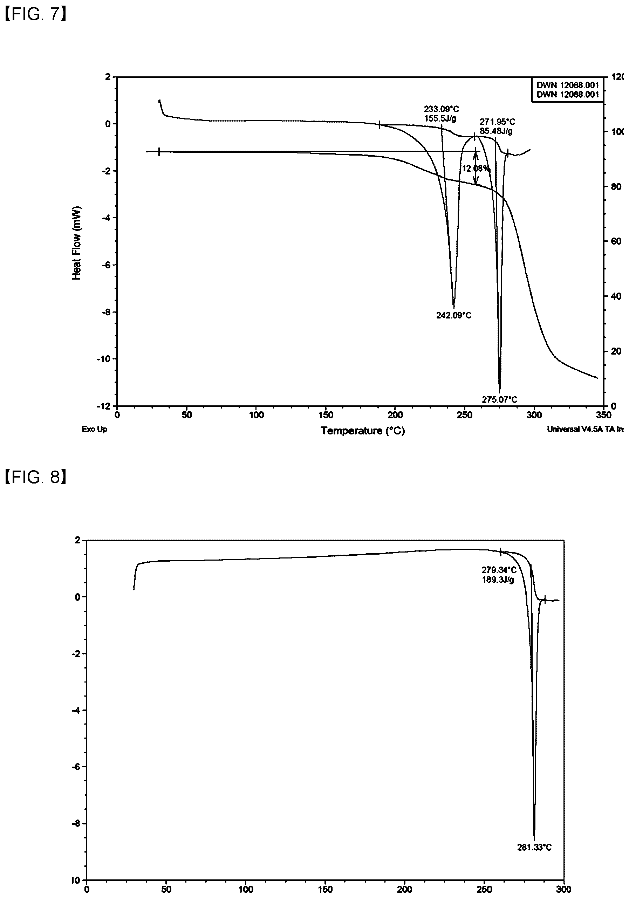

Referring to FIG. 7, it can be confirmed that (2R, 3S)-2-(3-(4,5-dichloro-1H-benzo[d]imidazol-1-yl)propyl)piperidin-3-ol dihydrochloride prepared in Comparative Example 1 shows two endothermic peaks (a first peak having an endothermic onset temperature of 233.09°C and an endothermic temperature of the maximum endothermic peak within the peak of 242.09°C and a second peak having an endothermic onset temperature of 271.95°C and an endothermic peak of 275.07°C). In addition, residual solvent and moisture were not confirmed by thermogravimetric analysis.

### Test Example 3: Hygroscopicity test

The hygroscopicity tests of the acid addition salts prepared in Examples 1-1 to 1-6 and Comparative Example 1 were performed.

40 mg of each acid addition salt sample was placed in a 10 mL beaker, and this was placed in each glass desiccator containing a saturated aqueous solution of several salts under conditions of constant relative humidity at room temperature as shown in Table 1 below, and stored in a tightly closed state. After one day, the change in weight was measured, then transferred to another desiccator and the previous experiment was repeated. At this time, for sorption analysis, the weight change was measured by moving the sample from a low-humidity desiccator to a high-humidity desiccator, and for desorption analysis, the weight change was measured by transferring the sample from a high-humidity desiccator with high humidity to a low-humidity desiccator. The results are shown in Table 2 below.

**[Table 1]**

| Desiccator | Relative humidity | Type of salt saturated aqueous solution |
|---|---|---|
| 1 | 11% RH | LiCl₂ saturated aqueous solution |
| 2 | 33% RH | MgCl₂ saturated aqueous solution |
| 3 | 54% RH | Mg(NO₃)₂ · 6H₂O saturated aqueous solution |
| 4 | 75% RH | NaCl saturated aqueous solution |
| 5 | 93% RH | KNOs saturated aqueous solution |

**[Table 2]**

| | | Example 1-1 | Example 1-2 | Example 1-3 | Example 1-4 | Example 1-5 | Example 1-6 | Comparat ive Example 1 |
|---|---|---|---|---|---|---|---|---|
| Type of acid addition salt | | Mono-hydrochlo ride | di-oxalate I | di-oxalate II | hemi-oxalate | hemi-maleate | monopalmitate | di-hydrochlo ride |
| Sorpt ion | 11% RH | 100 | 100 | 100 | 100 | 100 | 100 | 100 |
| | 33% RH | 100 | 101.1 | 100 | 101.0 | 100 | 100 | 100.9 |
| | 54% RH | 101 | 101.8 | 100 | 101.6 | 100.7 | 100.7 | 108 |
| | 75% RH | 101.6 | 102.9 | 101.3 | 101.9 | 100.9 | 100.7 | 105.4 |
| | 93% RH | 101.6 | 105.6 | 101.3 | 105.4 | 101.6 | 102.3 | 128 |
| Desc rptio n | 75% RH | 101.7 | 102.9 | 100.8 | 103.2 | 100.5 | 100.7 | 105.4 |
| | 54% RH | 100.8 | 102 | 100.2 | 101.9 | 99.8 | 100 | 104.7 |
| | 33% RH | 101.2 | 101.6 | 100 | 101.0 | 99.8 | 100 | 103.9 |
| | 11% RH | 100.8 | 100 | 100 | 101.0 | 99.5 | 100 | 102.9 |

Referring to Table 2, it can be seen that the acid addition salts of Examples not only has little change in weight due to moisture even with an increase in relative humidity, but also show significantly lower hygroscopicity at 93% RH compared to the dihydrochloride of Comparative Example 1.

### Test Example 4: Stability Confirmation Test

The acid addition salts prepared in some Examples and Comparative Examples were subjected to stability tests against high temperature and high humidity.

Specifically, the severe tests with respect to temperature were performed by placing 50 mg of a sample in a vial, closing the lid, and then measuring the content of impurities using HPLC (Azura, KNAUER, P6.1 1L, DAD 6.1L) after 1 week, 2 weeks, 4 weeks, and 8 weeks while storing in a chamber at 60°C and a relative humidity of less than 10%.

And, the severe tests with respect to moisture were performed by placing 50 mg of a sample in a 10 mL beaker, and measuring the content of impurities using HPLC (Azura, KNAUER, P6.1 1L, DAD 6.1L) after 1 week, 2 weeks, 4 weeks, and 8 weeks while storing the sample in a 93% RH desiccator in a state that the lid was not closed so that the sample has sufficient contact with the moisture in the air.

### (1) Calculation of the peak area of the main component using HPLC

At a fixed time after the start of the test, one vial or beaker was taken out from each time point (the number of samples per test: n=1), and impurities were analyzed using HPLC under the conditions shown in Table 3 below. Then, the peak area was determined through integration for all the detected peaks, and then the relative peak area (%) for the main component was calculated and expressed as an average value. The results are shown in Table 4 below.

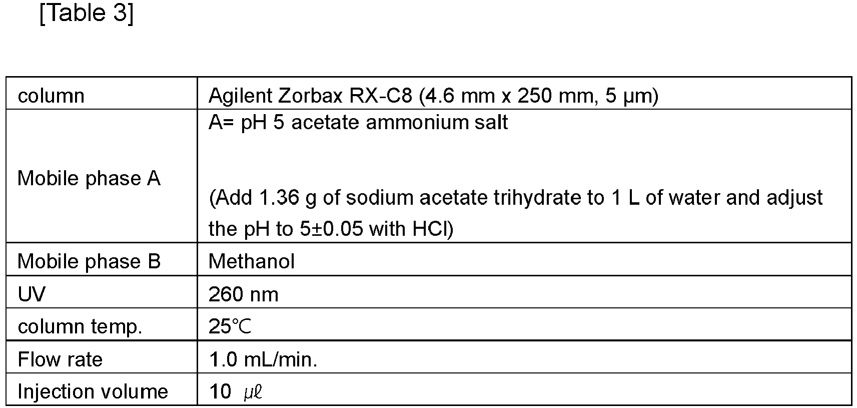

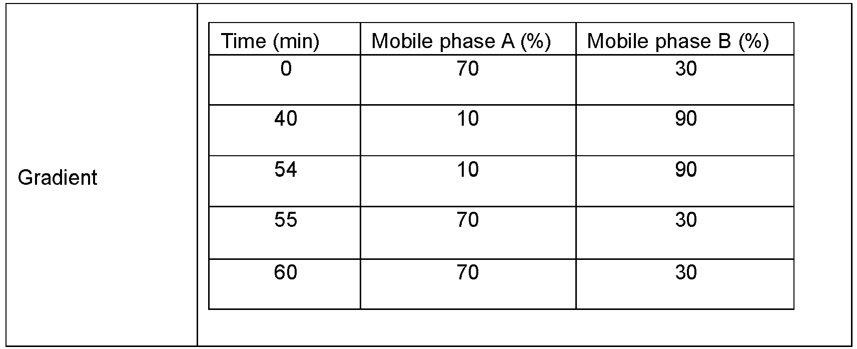

**[Table 4]**

| | | Example 1-1 | Example 1-2 | Example 1-3 | Example 1-5 | Comparative Example 1* |
|---|---|---|---|---|---|---|
| Type of acid addition salt | | Monohydrochloride | dioxalate I | dioxalate II | hemi-maleate | dihydrochloride |
| 60°C | initial | 99.9 | 99.8 | 99.8 | 99.8 | 99.6 |
| | after 1 week | 99.9 | 99.9 | 99.9 | - | 99.7 |
| | after 2 weeks | 99.8 | 99.8 | 99.8 | 99.8 | 99.7 |
| | after 4 weeks | 99.9 | 99.7 | 99.8 | 99.7 | 99.8 |
| | after 8 weeks | 99.8 | 99.8 | 99.9 | 99.9 | 99.7 |
| 93% RH | initial | 99.9 | 99.8 | 99.8 | 99.8 | 99.6 |
| | after 1 week | 99.8 | 99.3 | 99.7 | - | 99.7 |
| | after 2 weeks | 99.9 | 99.7 | 99.8 | 99.8 | 99.7 |
| | after 4 weeks | 99.8 | 99.8 | 99.9 | 99.8 | 99.7 |
| | after 8 weeks | 99.9 | 99.8 | 99.9 | 99.9 | 99.7 |

| | | | | | | |
|---|---|---|---|---|---|---|
| (However, dihydrochloride deliquesces within 1 week under 93% RH condition) | | | | | | |

As shown in Tables 3 and 4, it can be seen that the acid addition salts prepared in Examples did not show a significant decrease in the peak area of the main component under high temperature conditions and high humidity exposure conditions. Therefore, it can be confirmed that the crystalline forms prepared in Examples suppress the increase in impurities independently of the influence of temperature and humidity under severe conditions, thus exhibiting excellent chemical stability.

### (2) Differential scanning calorimetry analysis

For (2R, 3S)-2-(3-(4,5-dichloro-1H-benzo[d]imidazol-1-yl)propyl)piperidin-3-ol monohydrochloride prepared in Example 1-1 and (2R, 3S)-2-(3-(4,5-dichloro-1H-benzo[d]imidazol-1-yl)propyl)piperidin-3-ol dihydrochloride prepared in Comparative Example 1, each beaker was taken out 1 week and 8 weeks after the start of the test from the desiccator where the severe tests with respect to temperature were in progress, and the differential scanning calorimetry analysis was performed. The results are shown in FIGS. 8 to 11, respectively. At this time, the differential scanning calorimetry analysis was performed using a differential scanning calorimeter (DSC Q20, manufactured by TA Instruments) in a tightly closed pan under nitrogen purge at a scan rate of 10°C/min while raising the temperature from 20°C to 300°C.

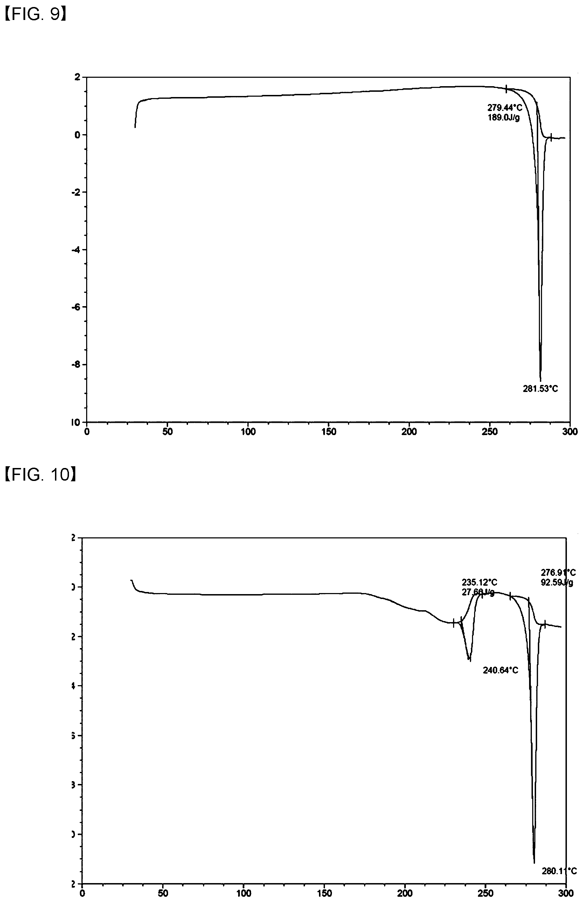

Specifically, FIGS. 8 and 9 each show the results of differential scanning calorimetry analysis after 1 week and 8 weeks of severe tests with respect to temperature of (2R, 3S)-2-(3-(4,5-dichloro-1H-benzo[d]imidazol-1-yl)propyl)piperidin-3-ol monohydrochloride prepared in Example 1-1.

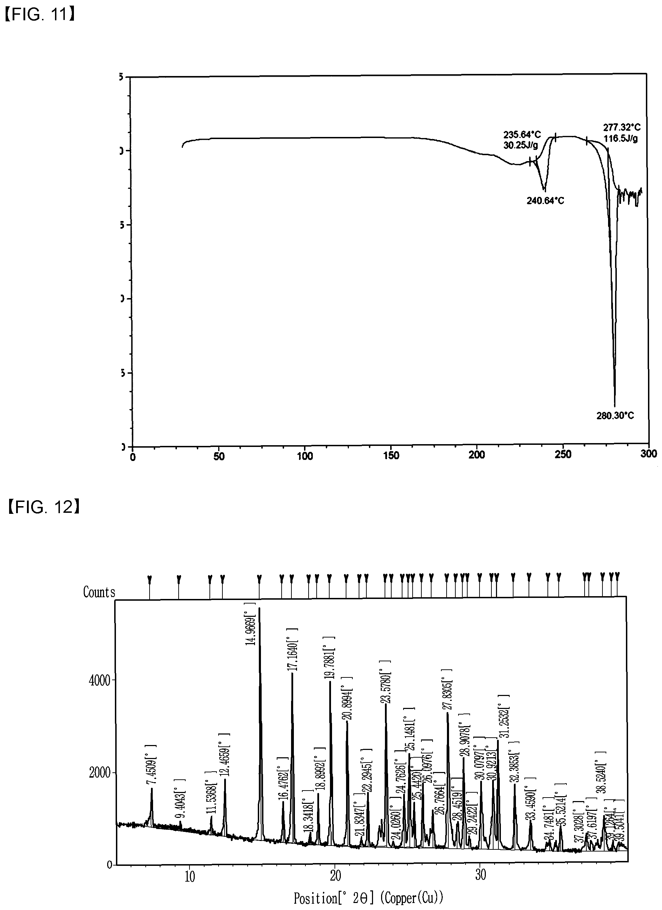

Specifically, FIGS. 10 and 11 each show the results of differential scanning calorimetry analysis after 1 week and 8 weeks of severe tests with respect to temperature of (2R, 3S)-2-(3-(4,5-dichloro-1H-benzo[d]imidazol-1-yl)propyl)piperidin-3-ol dihydrochloride prepared in Comparative Example 1.

Referring to FIGS. 8 to 11, it can be seen that (2R, 3S)-2-(3-(4,5-dichloro-1H-benzo[d]imidazol-1-yl)propyl)piperidin-3-ol dihydrochloride prepared in Comparative Example 1 shows a change in the differential scanning calorimetry analysis pattern while the first peak in the front portion becomes smaller with the lapse of time under a high temperature condition, whereas (2R, 3S)-2-(3-(4,5-dichloro-1H-benzo[d]imidazol-1-yl)propyl)piperidin-3-ol monohydrochloride prepared in Example 1-1 does not show any change in differential scanning calorimetry analysis pattern even after the lapse of 8 weeks under high-temperature conditions. Through this, it was confirmed that (2R, 3S)-2-(3-(4,5-dichloro-1H-benzo[d]imidazol-1-yl)propyl)piperidin-3-ol monohydrochloride has excellent chemical stability.

### Example 2-1: Preparation of crystalline form of (2R, 3S)-2-(3-(4,5-dichloro-1H-benzo[d]imidazol-1-yl)propyl)piperidin-3-ol monohydrochloride using acetonitrile solvent

10 mg of (2R, 3S)-2-(3-(4,5-dichloro-1H-benzo[d]imidazol-1-yl)propyl)piperidin-3-ol monohydrochloride prepared in Example 1-1 was suspended in 1 mL of acetonitrile solvent, and then stirred at room temperature for 3 hours. Then, the prepared suspension was left at room temperature for at least one day to evaporate the solvent. After crystal formation, the crystals were separated through vacuum filtration to obtain 8 mg of crystalline form I of (2R, 3S)-2-(3-(4,5-dichloro-1H-benzo[d]imidazol-1-yl)propyl)piperidin-3-ol monohydrochloride.

### Examples 2-2 to 2-9

(2R, 3S)-2-(3-(4,5-dichloro-1H-benzo[d]imidazol-1 -yl)propyl)piperidin-3-olyl crystals of hydrochloride were obtained in the same manner as in Example 2-1, except that in Example 2-1, the solvent shown in Table 5 below was used instead of the acetonitrile solvent. As a result of differential scanning calorimetry analysis, it can be seen that the prepared crystal shows the same crystalline form (crystalline form I) as in Example 2-1.

### Example 2-10

10 mg of (2R, 3S)-2-(3-(4,5-dichloro-1H-benzo[d]imidazol-1-yl)propyl)piperidin-3-ol monohydrochloride prepared in Example 1-1 was dissolved in 1 mL of methanol solvent and then stirred at room temperature for 3 hours. Then, the prepared solution was left at room temperature for at least one day to evaporate the solvent. After crystal formation, crystals were obtained through vacuum filtration, and as a result of differential scanning calorimetry analysis, it can be seen that the prepared crystal shows the same crystalline form (crystallin form I) as in Example 2-1.

### Example 2-11

10 mg of (2R, 3S)-2-(3-(4,5-dichloro-1H-benzo[d]imidazol-1-yl)propyl)piperidin-3-ol monohydrochloride prepared in Example 1-1 was dissolved in 0.8 mL of methanol solvent to prepare a solution. Then, 1 mL of acetonitrile solvent as a non-solvent was added to the prepared solution, and left at room temperature for at least one day. After crystal formation, crystals were obtained through vacuum filtration, and as a result of differential scanning calorimetry analysis, it can be seen that the prepared crystal shows the same crystalline form (crystalline form I) as in Example 2-1.

### Examples 2-12 to 2-19

A crystalline form of (2R, 3S)-2-(3-(4,5-dichloro-1H-benzo[d]imidazol-1-yl)propyl)piperidin-3-ol monohydrochloride were obtained in the same manner as in Example 2-11, except that in Example 2-11, the solvent shown in Table 5 below was used instead of acetonitrile as a non-solvent. As a result of differential scanning calorimetry analysis, it can be seen that the prepared crystal shows the same crystalline form (crystallin form I) as in Example 2-1.

**[Table 5]**

| | Solvent | Type of Crystalline form | | Solvent/non-solvent | Type of crystalline form |
|---|---|---|---|---|---|
| Example 2-1 | Acetonitrile | Crystalline form I | Example 2-11 | methanol /acetonitrile | Crystalline form I |
| Example 2-2 | Acetone | Crystalline form I | Example 2-12 | methanol/acetone | Crystalline form I |
| Example 2-3 | Ethyl acetate | Crystalline form I | Example 2-13 | methanol/ethyl acetate | Crystalline form I |
| Example 2-4 | Tetrahydrofuran | Crystalline form I | Example 2-14 | methanol/tetrahydrofuran | Crystalline form I |
| Example 2-5 | Isopropyl alcohol | Crystalline form I | Example 2-15 | methanol/isopropyl alcohol | Crystalline form I |
| Example 2-6 | Dichloromethane | Crystalline form I | Example 2-16 | methanol/dichloromethane | Crystalline form I |
| Example 2-7 | Ethyl ether | Crystalline form I | Example 2-17 | methanol/ethyl ether | Crystalline form I |
| Example 2-8 | Toluene | Crystalline form I | Example 2-18 | methanol/toluene | Crystalline form I |
| Example 2-9 | Hexane | Crystalline form I | Example 2-19 | methanol/hexane | Crystalline form I |
| Example 2-10 | Methanol | Crystalline form I | | | |

Referring to Table 5, it can be confirmed that (2R, 3S)-2-(3-(4,5-dichloro-1H-benzo[d]imidazol-1-yl)propyl)piperidin-3-ol monohydrochloride produces only one crystalline form even if the crystallization method and the solvent used are different. Particularly, the (2R, 3S)-2-(3-(4,5-dichloro-1H-benzo[d]imidazol-1-yl)propyl)piperidin-3-ol monohydrochloride prepared under the methanol/acetone solvent conditions of Example 2-12 is a single crystal.

### Comparative Example 2: Preparation of the crystalline form of (2R, 3S)-2-(3-(4,5-dichloro-1H-benzo[d]imidazol-1-yl)propyl)piperidin-3-ol dihydrochloride using acetonitrile solvent

Since (2R, 3S)-2-(3-(4,5-dichloro-1H-benzo[d]imidazol-1-yl)propyl)piperidin-3-ol dihydrochloride prepared in Comparative Example 1 is a crystalline form, this was used as Comparative Example 2.

### Test Example 5: X-ray powder diffraction analysis

The crystalline forms prepared in Example 2-1 and Comparative Example 2 were subjected to X-ray powder diffraction analysis, and the results are shown in FIGS. 1 and 2, respectively. At this time, the X-ray powder diffraction analysis was performed in the range of diffraction angles (2θ) from 5° to 35° using a CuKα target and an X-ray powder diffraction spectrometer (D8 Advance, manufactured by Bruker) under the conditions of a voltage of 45 kV, an amount of current of 40 mA, a divergence and scattering slit of 1°, a receiving slit of 0.2 mm, and a scanning speed of 3°/min (0.4 sec/0.02° interval).

Referring to FIG. 12, it can be confirmed that the crystalline form of (2R, 3S)-2-(3-(4,5-dichloro-1H-benzo[d]imidazol-1-yl)propyl)piperidin-3-ol monohydrochloride prepared in Comparative Example 2-1 has peaks at diffraction angles (2θ) of 7.5°, 12.5°, 15.0°, 16.5°, 17.2°, 18.9°, 19.8°, 20.9°, 22.3°, 23.6°, 24.8°, 25.1°, 26.1°, 27.8°, 28.9°, 30.1°, 30.9°, 31.3° and 32.4° in the X-ray powder diffraction pattern.

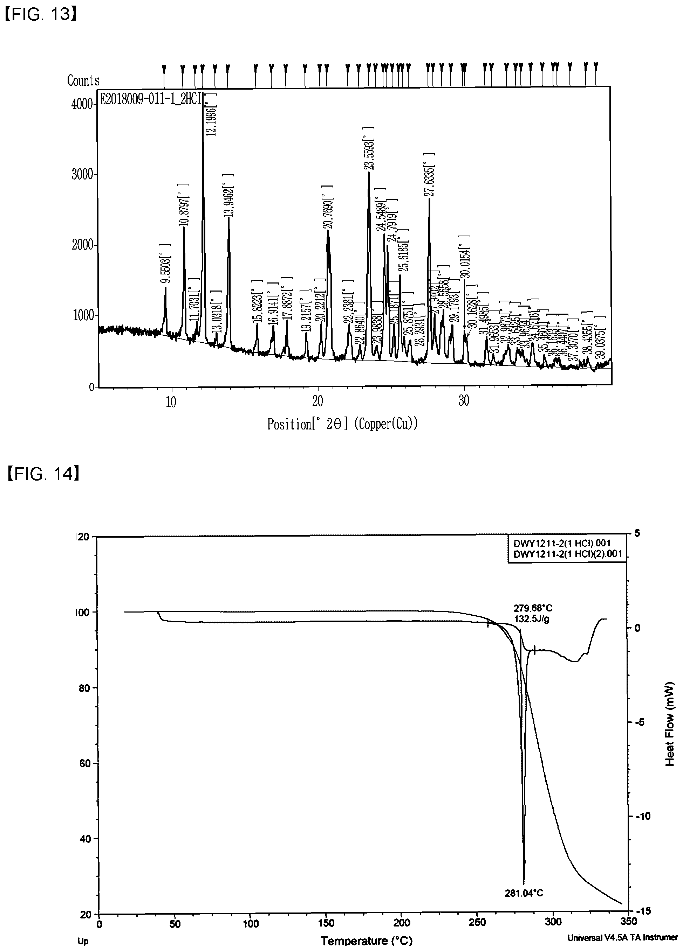

Referring to FIG. 13, it can be confirmed that the crystalline form of (2R, 3S)-2-(3-(4,5-dichloro-1H-benzo[d]imidazol-1-yl)propyl)piperidin-3-ol dihydrochloride prepared in Comparative Example 2 has peaks at diffraction angles (2θ) of 9.6°, 10.9°, 12.2°, 13.9°, 15.8°, 16.9°, 17.9°, 19.2°, 20.2°, 20.8°, 22.2°, 23.6°, 24.5°, 24.8°, 25.2°, 25.6°, 27.6°, 27.9°, 28.6°, 29.2°, 30.2° and 31.5° in the X-ray powder diffraction pattern.

Further, referring to FIGS. 12 and 13, it can be seen that the crystalline form of (2R, 3S)-2-(3-(4,5-dichloro-1H-benzo[d]imidazol-1-yl)propyl)piperidin-3-ol monohydrochloride prepared in Example 2-1 and the crystalline form of (2R, 3S)-2-(3-(4,5-dichloro-1H-benzo[d]imidazol-1-yl)propyl)piperidin-3-ol dihydrochloride prepared in Comparative Example 2 are different crystalline forms from each other.

### Test Example 6: Differential scanning calorimetry analysis of crystalline form

The crystalline form prepared in Example 2-1 was subjected to differential scanning calorimetry analysis, and the results are shown in FIG. 14. At this time, the differential scanning calorimetry analysis was performed using a thermogravimetric analyzer (DSC Q20, manufactured by TA Instruments) in a tightly closed pan at a scan rate of 10°C/min under nitrogen purge while raising the temperature from 20°C to 300°C.

Referring to FIG. 14, it can be confirmed that the crystalline form of (2R, 3S)-2-(3-(4,5-dichloro-1H-benzo[d]imidazol-1-yl)propyl)piperidin-3-ol monohydrochloride prepared in Example 2-1 has an endothermic onset temperature of 279.68 ±3°C and shows a maximum endothermic peak at the endothermic temperature of 281.04 ±3°C in differential scanning calorimetry analysis. Therefore, the crystalline form of (2R, 3S)-2-(3-(4,5-dichloro-1H-benzo[d]imidazol-1-yl)propyl)piperidin-3-ol monohydrochloride prepared in Example 2-1 not only has a higher endothermic onset temperature but also a higher endothermic temperature with a maximum endothermic peak than those of the crystalline form of the dihydrochloride salt, and thus can be improved in thermal stability as compared to the crystalline form of the dihydrochloride.

In addition, the results of differential scanning calorimetry analysis of the crystalline form of (2R, 3S)-2-(3-(4,5-dichloro-1H-benzo[d]imidazol-1-yl)propyl)piperidin-3-ol monohydrochloride were the same as the results of differential scanning calorimetry analysis of the crystalline form of (2R, 3S)-2-(3-(4,5-dichloro-1H-benzo[d]imidazol-1-yl)propyl)piperidin-3-ol monohydrochloride (crystalline form I). Therefore, it can be seen that the monohydrochloride prepared in Example 1-1 is also a crystalline form, and the crystal form thereof is the same as the crystalline form (crystalline form I) of the monohydrochloride prepared in Example 2-1.

## Claims

1. A (2R, 3S)-2-(3-(4,5-dichloro-1H-benzo[d]imidazol-1-yl)propyl)piperidin-3-ol monohydrochloride.

2. The (2R, 3S)-2-(3-(4,5-dichloro-1H-benzo[d]imidazol-1-yl)propyl)piperidin-3-ol monohydrochloride according to claim 1,
wherein the monohydrochloride has an endothermic onset temperature of 279.68 ± 3°C and shows a maximum endothermic peak at the endothermic temperature of 281.04 ±3°C in differential scanning calorimetry analysis.

3. An acid addition salt of (2R, 3S)-2-(3-(4,5-dichloro-1H-benzo[d]imidazol-1-yl)propyl)piperidin-3-ol, wherein the acid addition salt is oxalate, maleate, or palmitate.

4. The acid addition salt of (2R, 3S)-2-(3-(4,5-dichloro-1H-benzo[d]imidazol-1 -yl)propyl)piperidin-3-ol according to claim 3,
wherein the acid addition salt is dioxalate.

5. The acid addition salt of (2R, 3S)-2-(3-(4,5-dichloro-1H-benzo[d]imidazol-1-yl)propyl)piperidin-3-ol according to claim 4,
wherein the dioxalate has an endothermic onset temperature of 210.42 ±3°C and shows a maximum endothermic peak at the endothermic temperature of 213.66 ±3°C in differential scanning calorimetry analysis.

6. The acid addition salt of (2R, 3S)-2-(3-(4,5-dichloro-1H-benzo[d]imidazol-1-yl)propyl)piperidin-3-ol according to claim 4,
wherein the dioxalate has an endothermic onset temperature of 193.84 ±3°C and shows a maximum endothermic peak at the endothermic temperature of 203.92 ±3°C in differential scanning calorimetry analysis.

7. The acid addition salt of (2R, 3S)-2-(3-(4,5-dichloro-1H-benzo[d]imidazol-1 -yl)propyl)piperidin-3-ol according to claim 3,
wherein, the acid addition salt is hemi-oxalate.

8. The acid addition salt of (2R, 3S)-2-(3-(4,5-dichloro-1H-benzo[d]imidazol-1-yl)propyl)piperidin-3-ol according to claim 7,
wherein the hemi-oxalate has an endothermic onset temperature of 253.72 ±3°C and shows a maximum endothermic peak at the endothermic temperature of 255.62 ±3°C in differential scanning calorimetry analysis.

9. The acid addition salt of (2R, 3S)-2-(3-(4,5-dichloro-1H-benzo[d]imidazol-1-yl)propyl)piperidin-3-ol according to claim 3,
wherein the acid addition salt is hemi-maleate.

10. The acid addition salt of (2R, 3S)-2-(3-(4,5-dichloro-1H-benzo[d]imidazol-1-yl)propyl)piperidin-3-ol according to claim 9,
wherein the hemi-maleate has an endothermic onset temperature of 90.69±3°Cand shows a maximum endothermic peak at the endothermic temperature of 110.16±3°C in differential scanning calorimetry analysis.

11. The acid addition salt of (2R, 3S)-2-(3-(4,5-dichloro-1H-benzo[d]imidazol-1-yl)propyl)piperidin-3-ol according to claim 3,
wherein the acid addition salt is palmitate.

12. The acid addition salt of (2R, 3S)-2-(3-(4,5-dichloro-1H-benzo[d]imidazol-1-yl)propyl)piperidin-3-ol according to claim 11,
wherein the palmitate has an endothermic onset temperature of 92.32±3°C and shows a maximum endothermic peak at the endothermic temperature of 98.59±3°C in differential scanning calorimetry analysis.

13. A crystalline form of (2R, 3S)-2-(3-(4,5-dichloro-1H-benzo[d]imidazol-1-yl)propyl)piperidin-3-ol monohydrochloride, having peaks at diffraction angles (2θ±0.2°) of 15.0°, 17.2°, 19.8°, 23.6° and 27.8° in an X-ray powder diffraction pattern.

14. The crystalline form of (2R, 3S)-2-(3-(4,5-dichloro-1H-benzo[d]imidazol-1-yl)propyl)piperidin-3-ol monohydrochloride according to claim 13, having an endothermic onset temperature of 279.68 ±3°C and showing a maximum endothermic peak at the endothermic temperature of 281.04 ±3°C in differential scanning calorimetry analysis.

15. A pharmaceutical composition for preventing or treating cancer, inflammatory disease, autoimmune disease, or fibrosis, comprising at least one selected from the group consisting of (2R, 3S)-2-(3-(4,5-dichloro-1H-benzo[d]imidazol-1-yl)propyl)piperidin-3-ol monohydrochloride, (2R, 3S)-2-(3-(4,5-dichloro-1H-benzo[d]imidazol-1-yl)propyl)piperidin-3-ol oxalate, (2R, 3S)-2-(3-(4,5-dichloro-1H-benzo[d]imidazol-1-yl)propyl)piperidin-3-ol maleate, (2R, 3S)-2-(3-(4,5-dichloro-1H-benzo[d]imidazol-1-yl)propyl)piperidin-3-ol palmitate, and a crystalline form of (2R, 3S)-2-(3-(4,5-dichloro-1H-benzo[d]imidazol-1-yl)propyl)piperidin-3-ol monohydrochloride.
